# EUROPÄISCHE PATENTSCHRIFT

(11) **EP 2 553 083 B1**
(45) Veröffentlichungstag und Bekanntmachung des Hinweises auf die Patenterteilung: **06.03.2019**
(21) Anmeldenummer: 11712815.7
(22) Anmeldetag: 30.03.2011
(51) Int. Cl.: C12M 1/107

(54) **REGELUNG VON BIOGASANLAGEN**
CONTROL OF BIOGAS PLANTS
RÉGLAGE D'INSTALLATIONS DE BIOGAZ

(30) Priorität: 01.04.2010 DE 102010014240
(43) Veröffentlichungstag der Anmeldung: 06.02.2013
(73) Patentinhaber: KSB SE & Co. KGaA, 67227 Frankenthal (DE)
(72) Erfinder: ROSTALSKI, Kai, 06217 Merseburg (DE); SPRINGER, Peer, 67141 Neuhofen (DE)
(86) Internationale Anmeldenummer: PCT/EP2011/054909
(87) Internationale Veröffentlichungsnummer: WO 2011/121022

(56) Entgegenhaltungen:
- EP-A1- 0 516 895
- EP-A1- 1 762 607
- WO-A2-2008/104320
- DE-A1- 10 056 338
- DE-U1-202007 002 835

## Beschreibung

Die Erfindung betrifft ein Verfahren und eine Vorrichtung zur Herstellung von Biogas aus organischen Stoffen, wobei einem Behälter Substrat mittels eines Aufgabesystems zugeführt wird und in dem Behälter mindestens ein Rührwerk angeordnet ist.

Das Verfahren dient der Erzeugung von Biogas aus organischen Stoffen. Der in solchen Anlagen zur Biogaserzeugung eingesetzte Rohstoff wird als Substrat bezeichnet. Das Substrat besteht aus biogen abbaubarer Biomasse wie Gülle, Silage oder Bioabfall. Die zur Biogasproduktion eingesetzten Behälter werden auch als Bioreaktoren oder Fermenter bezeichnet. Im kontinuierlichen Betrieb der Biogasanlagen wird dem Behälter fortlaufend Substrat zugeführt und Biogas sowie Gärrest entnommen. Im Behälter befindliches Substrat wird durch verschiedene Arten von Mikroorganismen umgesetzt. Diese umzusetzende Biomasse wird als Gärsubstrat bezeichnet. Aufgrund des mikrobiellen Abbaus entstehen aus dem Gärsubstrat Methan und Kohlendioxid als Hauptkomponenten des Biogases.

Das zugeführte Substrat wird mit dem Behälterinhalt vermischt. Die Zuführung des Substrates erfolgt meist durch punktförmige Aufgabe mit Hilfe von Fütterungssystemen. Die für eine möglichst hohe Ausbeute an Biogas benötigte Verweilzeit der Biomasse ist entscheidend von der Vermischung des Substrats mit dem Gärsubstrat abhängig. Bei solchen Medien, die überwiegend durch erhöhte Viskositäten gekennzeichnet sind, ist zur Vermischung und/oder Durchmischung eine Umwälzung des Behälterinhalts erforderlich, was in der Regel mit Rührwerken erfolgt.

Im Bereich der anaeroben Bioreaktionstechnik werden bei vielen Anwendungen Fermenter mit einem Höhe zu Durchmesser Verhältnis von größer als 0,5 eingesetzt. Die Vermischung wird dabei meist mit Vertikal-Rührwerken vorgenommen. Dabei befinden sich die Rührwerkspropeller auf einer zentralen von außen angetriebenen Welle. Die Antriebswelle ist vertikal angeordnet und ragt von oben in den Behälter hinein, wobei sie meist parallel zu den Behälterwänden verläuft. Ein solcher Fermenter ist beispielsweise durch die DE 199 47 339 A1 bekannt.

Stattdessen werden bei dem erfindungsgemäßen Verfahren zur Herstellung von Biogas Behälterformen eingesetzt, deren Höhe zu Durchmesser Verhältnis kleiner als 0,5 ist. Der Durchmesser der Behälter liegt vorzugsweise zwischen 16 und 40 Meter. Bei diesen Behälterabmessungen ist der Einsatz eines von außen angetriebenen zentralen Vertikal-Rührwerkes nicht mehr wirtschaftlich. Zur Vermischung des Behälterinhalts kommen vorwiegend in der Randzone des Behälters und darin angeordnete Rührwerke zum Einsatz, die eine zumeist horizontale Strömung des Mediums im Behälter erzeugen. Eine solche Anordnung ist beispielsweise durch die WO 2008/104320 A2 bekannt.

Die DE 100 56 338 A1 zeigt ein Verfahren und eine Vorrichtung zur Behandlung eines Mediums mit Mikroorganismen, insbesondere zur Behandlung von Abwasser mit Mikroorganismen.

Durch die DE 20 2007 002 835 U1 sind für die Durchmischung des Behälterinhalts mehrere Rührwerke bekannt, wobei zwei übereinander angeordnete Rührwerke einem einzelnen Rührwerk gegenüber angeordnet sind. Für einen hohen Wirkungsgrad des Fermentationsprozesses wird eine möglichst gleichmäßige Biomasseverteilung in der Fermenterflüssigkeit als erforderlich angesehen. Zusätzlich ist eine Füllstandsmessvorrichtung vorgesehen, mit der die Füllhöhe im Behälter erfasst und ein entsprechendes Füllstandsmesssignal als Höhenistwertsignal erzeugt wird. Ein Füllstandsmesssignal wird einer Steuereinrichtung zugeführt, die bei einer erfassten geringeren Füllhöhe einen Höhenstellmotor für das als Tauchmotorrührgerät ausgebildete Rührwerk so ansteuert, dass dieses abgesenkt wird und dessen Rührflügel dadurch weiter vollständig eingetaucht sind.

Die zur Biogaserzeugung eingesetzten Gärsubstrate haben in der Regel strukturviskose Fließeigenschaften. Strukturviskos bedeutet, dass mit zunehmender Schergeschwindigkeit die dynamische Viskosität vom Gärsubstrat abnimmt. Die Viskosität ist somit kein Wert sondern eine Funktion. Zu jeder induzierten Scherrate stellt sich eine dazugehörige Viskosität ein. Damit ist die Viskosität im Behälter örtlich unterschiedlich. Sie hängt von den lokal vorhandenen Scherraten ab. Ursächlich dafür sind die örtlichen Geschwindigkeiten, welche die Strömung im Behälter beeinflussen.

Scherraten werden durch die Bewegung des Propellers eines Rührwerkes erzeugt. In der Umgebung um den Propeller nimmt bei strukturviskosen Gärsubstraten die lokale Viskosität ab. Mit zunehmendem Abstand vom Propeller verringert sich die Schergeschwindigkeit und die Viskosität steigt entsprechend an. Dies führt dazu, dass der Propeller vorwiegend Gärsubstrat aus propellernahen Bereichen ansaugt, in denen das Gärsubstrat eine geringe Viskosität besitzt. Dadurch entstehen propellernahe Bereiche, in welchen das Substrat mit hohen Geschwindigkeiten in einem kleinen Volumen lediglich um den Propeller selbst herum transportiert wird. Diese propellernahen Bereiche werden als Kavernen bezeichnet. Arbeiten Rührwerke lediglich lokal in einer Kaverne findet keine optimale Durchmischung des Behälterinhalts statt, weil sich die Strömungserzeugung auf diese Bereiche beschränkt. Infolgedessen führt dies zur Verringerung des nutzbaren Reaktorvolumens bezogen auf den tatsächlichen Rauminhalt des Bioreaktors. Im Ergebnis wird im kleineren nutzbaren Reaktorvolumen weniger Biogas und damit auch weniger nutzbares Methan erzeugt. Der Methananteil oder die Methanmenge haben Auswirkungen auf den wirtschaftlichen Betrieb eines Bioreaktors.

Aufgabe der Erfindung ist die Maximierung der umgesetzten Gärsubstratmenge und der erzeugten Methangasmenge. Weiterhin soll die zur Biogasproduktion benötigte Verweildauer und der erforderliche Energiebedarf reduziert werden.

Diese Aufgabe wird erfindungsgemäß dadurch gelöst, dass der Istwert von mindestens einer Messgröße erfasst und an eine Regeleinheit weitergeleitet wird und in der Regeleinheit ein Sollwert hinterlegt ist, wobei die Regeleinheit die Abweichung des Istwerts vom Sollwert errechnet und in Abhängigkeit dieser Abweichung Stellgrößen variiert, welche den Leistungseintrag des Rührwerks und/oder die Zusammensetzung des Behälterinhalts und/oder das Fließverhalten des Behälterinhalts verändern, wobei als mindestens eine Messgröße das Fließverhalten des Gärsubstrats ermittelt wird, insbesondere die Bildung von Kavernen im propellernahen Bereich, und dass ein betriebsspezifischer Sollwert von der Größe des Behälters und/oder der Art der Rührwerke und/oder der Position der Rührwerke zueinander abhängt.

Die Wirtschaftlichkeit einer Biogasanlage ist wesentlich von dem Eigenenergieverbrauch der Rührwerke abhängig. Mit dem erfindungsgemäßen Verfahren wird die zu einem optimalen Betrieb der Anlage notwendige hydraulische Leistung minimiert. In der Regeleinheit werden Daten aus verschiedenen prozessüberwachenden Sensoren erfasst. Als Regeleinheit kann beispielsweise eine speicherprogrammierbare Steuerung eingesetzt werden. Je nach Programmierung der Regeleinheit können eine oder mehrere Messgrößen als Führungsgrößen zur Regelung des Rührwerks herangezogen werden.

Die Regeleinheit vergleicht die Messwerte mit anlagenspezifischen Sollwerten. Die betriebsspezifischen Sollwerte hängen beispielsweise von der Größe des Behälters, der Art der Rührwerke und der Anordnung der Rührwerke zueinander ab. Sie werden für jeden Anwendungsfall festgelegt.

In Abhängigkeit der Regeldifferenz variiert die Regeleinheit Stellgrößen, welche den Leistungseintrag des Rührwerks und/oder die Zusammensetzung des Behälterinhalts und/oder das Fließverhalten des Behälterinhalts verändern. Erfindungsgemäß können von der Regeleinheit unterschiedliche prozessbeeinflussende Aggregate geregelt werden. Dazu zählen vorzugsweise die Rührwerke, eine Fermenterheizung, ein Aufgabesystem und eine Rezirkulationseinheit.

In einer Biogasanlage ist die Methanerzeugung ein Hauptziel. Bei einer besonders vorteilhaften Variante der Erfindung wird der entstehende Gasmengenstrom als Messgröße erfasst und als Führungsgröße zur Regelung der prozessbeeinflussenden Aggregate verwendet. Dazu kann ein Gasmengenzähler eingesetzt werden. Sinkt die Gasmenge unter ein bestimmtes Niveau variiert die Regeleinheit beispielsweise den Leistungseintrag des Rührwerks.

Bei der Biogasproduktion entstehen neben Methan auch andere Gase, wie beispielsweise Kohlendioxid. Ziel ist es, den Methananteil in dem entstehenden Gasstrom zu maximieren. Bei einer besonders vorteilhaften Ausführung der Erfindung werden daher als Messdaten der Methangasanteil erfasst und als Führungsgröße zur Regelung der prozessbeeinflussenden Aggregate herangezogen. Der Methangasanteil im Gasstrom kann durch Analysegeräte bestimmt werden. Vorzugsweise werden dazu Analysegeräte eingesetzt, die auf Basis Infrarotabsorption arbeiten.

Eine weitere Variante den Prozess zur Biogasproduktion zu regeln besteht darin als Messgröße die Leistungsdaten einer das erzeugte Methangas verarbeitenden Maschine/Aggregats heranzuziehen. Wird das Methangas in einer Verbrennungsmaschine verbrannt, so kann die Leistung dieser Maschine als Führungsgröße zur Regelung von prozessbeeinflussenden Aggregaten verwendet werden.

Bei einem laufenden Betrieb können sich durch Anhaften von Gasblasen an Strukturmaterialien des Gärsubstrates Schwimmschichten ausbilden. Das Aufschwimmen dieser Strukturmaterialien und deren Verfestigung auf der Oberfläche des Gärsubstrates wirken sich negativ auf den Fermentationsprozess aus. In der Hauptsache kann eine gasundurchlässige Schwimmschicht entstehen, die einen Biogasaustritt be- oder verhindert. Dies führt zu einer negativen Beeinflussung des Umwandlungsprozesses im Gärsubstrat.

Bei einer besonders vorteilhaften Variante der Erfindung wird als Messgröße das Ausmaß einer sich auf dem Gärsubstrat bildenden Schwimmschicht erfasst. Das Ausmaß der Schwimmschicht kann über die Höhe und/oder die Dichte der Schwimmschicht bestimmt werden. Die Regeleinheit regelt in Abhängigkeit von der sich auf dem Gärsubstrat bildenden Schwimmschicht die prozessbeeinflussenden Aggregate.

Als günstig erweist es sich, wenn als Messgröße das Biogasrestpotential in einem Gärrest erfasst wird. Durch eine regelmäßige Auswertung des dem Behälter kontinuierlich oder zyklisch entnommenen Gärrestes wird der erfolgte Stoffumsatz im Gärsubstrat bestimmt. Wird ein Biogasrestpotential festgestellt, dann werden diese Messdaten von der Regeleinheit ausgewertet. In Abhängigkeit der Messdaten bestimmt die Regeleinheit die Zeitpunkte für ein Ein-, Ab- oder Zuschalten eines oder mehrerer Rührwerke. Zusätzlich oder alternativ kann auch die Betriebszeitdauer der Rührwerke von der Regeleinheit variiert werden.

Bei einer besonders bevorzugten Ausführung der Erfindung wird der Leistungseintrag der Rührwerke über die Drehzahl der Rührwerke variiert. Weichen die Istwerte der Messgrößen von ihren Sollwerten ab, so verändert die Regeleinheit die Drehzahl der Rührwerke in Abhängigkeit der Regelabweichung. Der Leistungseintrag durch die Rührwerke kann auch dadurch variiert werden, dass weitere Rührwerke zugeschaltet werden. Die Zuschaltung liefert einen zusätzlichen Leistungseintrag.

Die Regeleinheit kann auch Stellgrößen variieren, welche die Zusammensetzung des Behälterinhalts verändern. Dazu kann die Regeleinheit die Menge an zugeführtem Substrats erhöhen oder herabsetzen. Eine weitere Möglichkeit besteht darin, den Aufschluss des Gärsubstrats durch Enzymeinsatz zu verändern. Auch eine Verdünnung des Gärsubstrats mittels Gülle und/oder Rezirkulat sowie eine Veränderung der Fließeigenschaften des Gärsubstrats durch die Hinzufügung von chemischen oder biologischen Wirkmechanismen sind möglich. Dabei kann auch nur eine phasenweise Zuschaltung von einem oder mehreren Rührwerken erfolgen, beispielsweise bei einer zeitweiligen Änderung der Substratzusammensetzung oder der Gärsubstratzusammensetzung durch Rezirkulatzuführung.

Weitere Merkmale und Vorteile der Erfindung ergeben sich aus der Beschreibung von Ausführungsbeispielen, anhand von Zeichnungen und aus den Zeichnungen selbst.

Dabei zeigt
Fig. 1 eine perspektivische Ansicht eines Behälters zur Biogasherstellung,
Fig. 2 eine schematische Darstellung der Regelung einer Anlage zur Biogasherstellung.

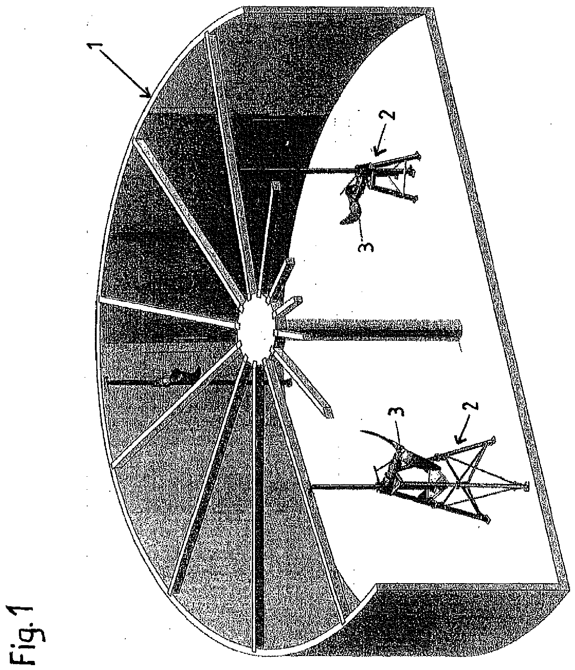

In Fig. 1 ist ein zylindrischer Behälter 1 zur Herstellung von Biogas dargestellt. Andere Behälterformen sind ebenfalls möglich. Das Verhältnis vom größten Durchmesser zur Höhe des Behälters ist kleiner 0,5. In dem Behälter 1 sind zwei Rührwerke 2 positioniert, deren Propeller 3 eine zu meist horizontale Strömung des Gärsubstrats im Behälter 1 erzeugen. Die Rührwerke 2 beziehungsweise deren Propeller 3 sind in unterschiedlichen Höhen innerhalb des Behälters 1 angeordnet. Ein Ergänzungsrührwerk 11 wird bei Bedarf dazugeschaltet.

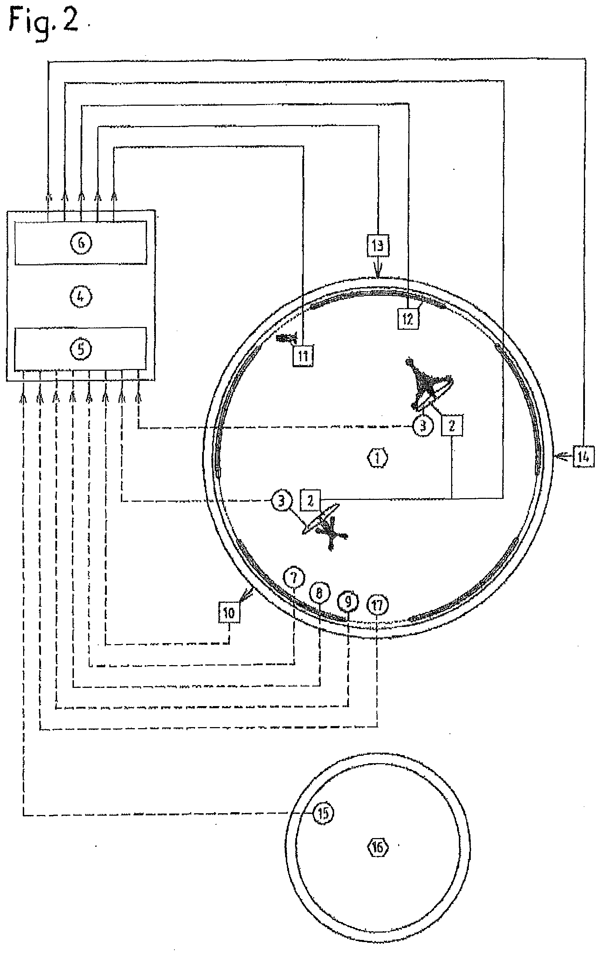

Fig. 2 zeigt eine schematisch dargestellte Regeleinheit 4 für ein Verfahren zur Biogasherstellung. Dabei kann es sich um eine speicherprogrammierbare Steuerung (SPS) oder ein anderes Regelsystem handeln.

Die Regeleinheit 4 verfügt über einen Signaleingangsbereich 5 und einen Signalsausgangsbereich 6. An den Signaleingangsbereich 5 werden die Signale der Daten geleitet, die bei Messungen am Verfahren erfasst werden. In der Regeleinheit 4 wer-den Daten aus verschiedenen prozessüberwachenden Sensoren 7, 8, 9, 10, 15, 17 und den Rührwerken 2 verarbeitet. Der Signalausgangsbereich 6 steht in Wirkverbin-dung mit prozessbeeinflussenden Aggregaten. Prozessbeeinflussende Aggregate sind die Rührwerke 2, 11, eine Fermenterheizung 12, ein Aufgabesystem 13 und eine Rezirkulationseinheit 14. Diese werden so gesteuert, dass sich einzelne Prozesspa-rameter mit dem Ziel eines maximalen Methanertrags optimieren lassen.

Folgende Sensoren können zur Prozessüberwachung eingesetzt werden: Mindestens ein Sensor zur Viskositätsmessung 7, ein oder mehreren Sensoren zur Fließgeschwindigkeitsmessung 8, mindestens ein Schwimmschichtdetektor 9, ein Gasmengenzähler 10, eine Einheit 15 zur Gärrestanalyse und mindestens eine Einheit 17 zur Fließverhaltenermittlung des Gärsubstrats.

Der Sensor 7 dient der Erfassung der Viskosität Zur Ermittlung der Viskosität können auch Messdaten, welche über die Rührwerke 2 ermittelt werden, herangezogen werden. Alternativ oder ergänzend ist der Einsatz einer separaten Fließverhaltenermittlungseinheit 17 möglich. Die Fließverhaltensermittlung kann dabei an mehreren Stel-len einzeln oder gleichzeitig erfolgen. Die Ermittlung des Fließverhaltens ist notwen-dig, um zu kritisches Fließverhalten im Behälter 1 hinsichtlich relevanter Prozesspa-rameter sowie Schäden an allen im Prozess eingesetzten Rührwerken 2, 11 zu ver-meiden und deren Eigenenergieverbrauch zu optimieren. Bei der Optimierung zwi-schen Gasertrag und Eigenenergieverbrauch ist die erzeugte Geschwindigkeit im Gärsubstrat von großer Bedeutung.

Der Sensor 8 wird zur Geschwindigkeitsmessung im Behälter 1 eingesetzt. Dabei kann die Geschwindigkeit mittels einer oder mehrerer Geschwindigkeitsermittlungen an unterschiedlichen Stellen erfolgen.

Mit einem Detektor 9 wird die Bildung einer Schwimmschicht überwacht. Da sich eine Schwimmschichtdecke nachteilig auf das Ausgasen des Biogases aus dem Gärsubstrat auswirkt, ist deren Entstehung zu vermeiden oder nach Entstehung möglichst früh zu zerstören. Dazu kann zum Beispiel ein Ergänzungsrührwerk 11 zugeschaltet und/oder ein oder mehrere Hauptrührwerke 2 können in ihrer Drehzahl variiert wer-den. Dadurch entsteht eine die Schwimmschicht auflösende Strömungsturbulenz.

Der erzeugte Gasmengenstrom wird vom einen Gasmengenzähler 10 erfasst. Sinkt die Gasmenge unter ein bestimmtes Niveau passt die Regeleinheit 4 den Leistungseintrag der Rührwerke 2, 11 an. Ziel der Fermentation ist es, eine möglichst große Menge des Biogaspotentials des Substrates zu nutzen.

Die Gärreste werden in einem Gärrestelager 16 gesammelt. Die Ermittlung des Biogasrestpotentials im Gärrest erfolgt durch die Einheit 15 und ist eine weitere mögliche Führungsgröße für die Regeleinheit 4 und die Regelung der Rührwerke 2, 11. Die Ermittlung des Biogasrestpotentials kann an unterschiedlichen Stellen der Anlage er-folgen. Ist im Gärrest ein bestimmtes Biogasrestpotential überschritten, passt die Re-geleinheit 4 die prozessbeeinflussenden Aggregate 2, 11, 12, 13, 14 an die Prozess-bedingungen an.

Grundsätzlich werden alle Daten aus dem Signaleingangsbereich 5 in der Regelein-heit 4 verarbeitet. Die Verarbeitung der Daten erfolgt auf Grundlage eines gespeicher-ten Algorithmus. Dieser Algorithmus übernimmt die Aufgabe, aus den Eingangsgrö-ßen die Werte für daraus bestimmte Regelgrößen zu ermitteln. Die ermittelten Regel-größen werden dazu benutzt, um vom Signalausgangsbereich 6 die den prozessbeeinflussenden Aggregate 2, 11, 12, 13, 14 zu steuern.

Von dem Signalausgangsbereich 6 gehen Signale zur Regelung unterschiedlicher Stellgrößen aus. Damit werden beispielsweise die Rührwerke 2 angesteuert, wobei deren Drehzahl geregelt werden kann.

Bei fehlender Bewegung an der Oberfläche des Gärsubstrats kann es zur Ausbildung einer Schwimmschicht kommen. Weiterhin kann eine fehlende Bewegung dazu füh-ren, dass das einzuspeisende Substrat oder das Gärsubstrat nur ungenügend im Be-hälter 1 verteilt werden.

Ein Ergänzungsrührwerk 11 kann bei der Zufuhr von neuem Substrat oder im Falle einer entstandenen Schwimmschicht dazugeschaltet oder geregelt werden. Die Hei-zung 12 führt bei der Einspeisung von neuem Substrat dem Behälter 1 Wärme zu. Das Substrat wird über das Aufgabesystem 13 zugeführt. Damit kann die Fütterungsmenge an die Prozessparameter angepasst werden. Eine Überfütterung des Behälters 1 mit Substrat würde sich nachteilig auf das Fließverhalten im Behälter 1 und damit auf die Methanproduktion auswirken. Verändert sich das Fließverhalten negativ, wird die Fütterungsmenge reduziert und/oder andere Regelgrößen, wie beispielsweise Geschwindigkeit oder Rezirkulatmenge, variiert.

Bei zu geringen Fütterungsmengen steht nicht genügend Substrat zur Methanbildung zur Verfügung. Mit Hilfe eines Gasmengenzähler 10 und/oder einer Analyse des Biogasrestpotentials im Gärrest wird dieser Zustand erkannt und eine Fütterung mit Substrat veranlasst. Das Verfahren verfügt über eine Rezirkulationseinheit 14 mit der es möglich ist Rezirkulat zuzudosieren. Auf diese Weise werden ein höherer Substratumsatz und eine gesteigerte Biogasausbeute erzielt.

## Patentansprüche

1. Verfahren zur Herstellung von Biogas aus organischen Stoffen, wobei einem Behälter (1) Substrat mittels eines Aufgabesystems (13) zugeführt wird und in dem Behälter (1) mindestens ein Rührwerk (2) angeordnet ist,
wobei ein Istwert von mindestens einer Messgröße erfasst und an eine Regeleinheit (4) weitergeleitet wird und in der Regeleinheit (4) ein Sollwert hinterlegt ist, wobei die Regeleinheit (4) die Abweichung des Istwerts vom Sollwert errechnet und in Abhängigkeit dieser Abweichung Stellgrößen variiert, welche den Leistungseintrag des Rührwerks (2) und/oder die Zusammensetzung des Behälterinhalts und/oder das Fließverhalten des Behälterinhalts verändern
**dadurch gekennzeichnet, dass**
als mindestens eine Messgröße das Fließverhalten des Gärsubstrats ermittelt wird, insbesondere die Bildung von Kavernen im propellernahen Bereich, und dass ein betriebsspezifischer Sollwert von der Größe des Behälters (1) und/oder der Art der Rührwerke und/oder der Position der Rührwerke zueinander abhängt.

2. Verfahren nach Anspruch 1, **dadurch gekennzeichnet, dass** als weitere Messgröße der entstehende Gasmengenstrom erfasst wird.

3. Verfahren nach Anspruch 1 oder 2, **dadurch gekennzeichnet, dass** als Messgröße der Methangasanteil erfasst wird.

4. Verfahren nach einem der Ansprüche 1 bis 3, **dadurch gekennzeichnet, dass** als Messgröße Leistungsdaten einer das Biogas verarbeitenden Maschine/Aggregats erfasst werden.

5. Verfahren nach einem der Ansprüche 1 bis 4, **dadurch gekennzeichnet, dass** als Messgröße das Ausmaß einer sich bildenden Schwimmschicht erfasst wird.

6. Verfahren nach einem der Ansprüche 1 bis 5, **dadurch gekennzeichnet, dass** als Messgröße das Biogasrestpotential in einem Gärrest erfasst wird.

7. Verfahren nach einem der Ansprüche 1 bis 6, **dadurch gekennzeichnet, dass** die Regeleinheit (4) die Drehzahl des Rührwerks (2) variiert.

8. Verfahren nach einem der Ansprüche 1 bis 7, **dadurch gekennzeichnet, dass** die Regeleinheit (4) mindestens ein zusätzliches Rührwerk (11) schaltet.

9. Verfahren nach einem der Ansprüche 1 bis 8, **dadurch gekennzeichnet, dass** die Regeleinheit (4) die Menge und/oder die Zusammensetzung des zugeführten Substrats und/oder eines Rezirkulats variiert und/oder die Fließeigenschaft des Gärsubstrats biochemisch oder physikalisch verändert.

10. Vorrichtung zur Herstellung von Biogas aus organischen Stoffen, wobei einem Behälter (1) Substrat mittels eines Aufgabesystems (13) zugeführt wird und in dem Behälter (1) mindestens ein Rührwerk (2) angeordnet ist, **dadurch gekennzeichnet, dass** der Istwert von mindestens einer Messgröße erfasst und an eine Regeleinheit (4) weitergeleitet wird und in der Regeleinheit (4) ein Sollwert hinterlegt ist, insbesondere ein betriebsspezifischer Sollwert, der von der Größe des Behälters (1) und/oder der Art der Rührwerke und/oder der Position der Rührwerke zueinander abhängt, wobei die Regeleinheit (4) die Abweichung des Istwerts vom Sollwert errechnet und in Abhängigkeit dieser Abweichung Stellgrößen variiert, welche den Leistungseintrag des Rührwerks (2) und/oder die Zusammensetzung des Behälterinhalts und/oder das Fließverhalten des Behälterinhalts verändern, insbesondere die Bildung von Kavernen im propellernahen Bereich verhindern.

11. Vorrichtung nach Anspruch 10, **dadurch gekennzeichnet, dass** als Messgröße der entstehende Gasmengenstrom erfasst wird.

12. Vorrichtung nach Anspruch 10 oder 11, **dadurch gekennzeichnet, dass** als Messgröße der Methangasanteil erfasst wird.

13. Vorrichtung nach einem der Ansprüche 10 bis 12, **dadurch gekennzeichnet, dass** als Messgröße die Leistungsdaten einer das Biogas verarbeitenden Maschine/Aggregats erfasst werden.

14. Vorrichtung nach einem der Ansprüche 10 bis 13, **dadurch gekennzeichnet, dass** als Messgröße das Ausmaß einer sich bildenden Schwimmschicht erfasst wird.

15. Vorrichtung nach einem der Ansprüche 10 bis 14, **dadurch gekennzeichnet, dass** als Messgröße das Biogasrestpotential in einem Gärrest erfasst wird.

16. Vorrichtung nach einem der Ansprüche 10 bis 15, **dadurch gekennzeichnet, dass** die Regeleinheit (4) die Drehzahl des Rührwerks (2) variiert.

17. Vorrichtung nach einem der Ansprüche 10 bis 16, **dadurch gekennzeichnet, dass** die Regeleinheit (4) mindestens ein zusätzliches Rührwerk (11) schaltet.

18. Vorrichtung nach einem der Ansprüche 10 bis 17, **dadurch gekennzeichnet, dass** die Regeleinheit (4) die Menge und/oder die Zusammensetzung des zugeführten Substrats und/oder eines Rezirkulats variiert und/oder die Fließeigenschaft des Gärsubstrats biochemisch oder physikalisch verändert.

## Claims

1. Method for producing biogas from organic substances, substrate being supplied to a container (1) by means of a feed system (13) and there being arranged in the container (1) at least one agitator mechanism (2), wherein an actual value of at least one measurement quantity is detected and transmitted to a regulating unit (4), and in the regulating unit (4) there is stored a setpoint value, wherein the regulating unit (4) calculates the deviation of the actual value from the setpoint value and, as a function of said deviation, varies manipulated quantities which vary the introduction of power by the agitator mechanism (2) and/or the composition of the container contents and/or the flow behavior of the container contents, **characterized in that** as at least one measurement variable, the flow behaviour of the fermentation substrate is determined, in particular the formation of caverns in the near-propeller region, and **in that** an operationally specific desired value depends on the size of the container (1) and/or on the type of agitator mechanisms and/or on the position of the agitator mechanisms in relation to one another.

2. Method according to Claim 1, **characterized in that** the gas mass flow generated is detected as a further measurement quantity.

3. Method according to Claim 1 or 2, **characterized in that** the methane gas fraction is detected as a measurement quantity.

4. Method according to one of Claims 1 to 3, **characterized in that** performance data of a machine/assembly which processes the biogas are detected as a measurement quantity.

5. Method according to one of Claims 1 to 4, **characterized in that** the extent of a floating layer which forms is detected as a measurement quantity.

6. Method according to one of Claims 1 to 5, **characterized in that** the biogas residue potential in a fermentation residue is detected as a measurement quantity.

7. Method according to one of Claims 1 to 6, **characterized in that** the regulating unit (4) varies the rotational speed of the agitator mechanism (2).

8. Method according to one of Claims 1 to 7, **characterized in that** the regulating unit (4) switches at least one additional agitator mechanism (11).

9. Method according to one of Claims 1 to 8, **characterized in that** the regulating unit (4) varies the quantity and/or the composition of the substrate supplied and/or of a recirculate and/or varies the flow property of the fermentation substrate biochemically or physically.

10. Appliance for producing biogas from organic substances, substrate being supplied to a container (1) by means of a feed system (13) and there being arranged in the container (1) at least one agitator mechanism (2), **characterized in that** the actual value of at least one measurement quantity is detected and transmitted to a regulating unit (4), and in the regulating unit (4) there is stored a setpoint value, in particular an operationally specific desired value, which is dependent on the size of the container (1) and/or on the type of agitator mechanisms and/or on the position of the agitator mechanisms in relation to one another, wherein the regulating unit (4) calculates the deviation of the actual value from the setpoint value and, as a function of said deviation, varies manipulated quantities which vary the introduction of power by the agitator mechanism (2) and/or the composition of the container contents and/or the flow behavior of the container contents, in particular prevent the formation of caverns in the near-propeller region.

11. Appliance according to Claim 10, **characterized in that** the gas mass flow generated is detected as a measurement quantity.

12. Appliance according to Claim 10 or 11, **characterized in that** the methane gas fraction is detected as a measurement quantity.

13. Appliance according to one of Claims 10 to 12, **characterized in that** the performance data of a machine/assembly which processes the biogas are detected as a measurement quantity.

14. Appliance according to one of Claims 10 to 13, **characterized in that** the extent of a floating layer which forms is detected as a measurement quantity.

15. Appliance according to one of Claims 10 to 14, **characterized in that** the biogas residue potential in a fermentation residue is detected as a measurement quantity.

16. Appliance according to one of claims 10 to 15, **characterized in that** the regulating unit (4) varies the rotational speed of the agitator mechanism (2).

17. Appliance according to one of claims 10 to 16, **characterized in that** the regulating unit (4) switches at least one additional agitator mechanism (11).

18. Appliance according to one of claims 10 to 17, **characterized in that** the regulating unit (4) varies the quantity and/or the composition of the substrate supplied and/or of a recirculate and/or varies the flow property of the fermentation substrate biochemically or physically.

## Revendications

1. Procédé de production de biogaz à partir de substances organiques, un substrat étant acheminé à un récipient (1) au moyen d'un système de distribution (13) et au moins un agitateur (2) étant disposé dans le récipient (1),
une valeur réelle d'au moins une grandeur mesurée étant acquise et retransmise à une unité de régulation (4) et une valeur de consigne étant stockée dans l'unité de régulation (4), l'unité de régulation .(4) calculant l'écart entre la valeur réelle et la valeur de consigne et, en fonction de cet écart, faisant varier des grandeurs de commande qui modifient la puissance consommée de l'agitateur (2) et/ou la composition du contenu du récipient et/ou l'aptitude à l'écoulement du contenu du récipient
**caractérisé en ce que**
l'aptitude à l'écoulement du substrat de fermentation est déterminée en tant qu'au moins une grandeur mesurée, notamment la formation de cavités dans la zone proche de l'hélice, et **en ce qu'**une valeur de consigne spécifique au fonctionnement dépend de la taille du récipient (1) et/ou de la nature des agitateurs et/ou de la position des agitateurs les uns par rapport aux autres.

2. Procédé selon la revendication 1, **caractérisé en ce que** le débit massique de gaz produit est acquis en tant que grandeur mesurée supplémentaire.

3. Procédé selon la revendication 1 ou 2, **caractérisé en ce que** la part de gaz méthane est acquise en tant que grandeur mesurée.

4. Procédé selon l'une des revendications 1 à 3, **caractérisé en ce que** des données de puissance d'une machine / d'un groupe qui traite le biogaz sont acquises en tant que grandeur mesurée.

5. Procédé selon l'une des revendications 1 à 4, **caractérisé en ce que** l'étendue d'une couche flottante qui se forme est acquise en tant que grandeur mesurée.

6. Procédé selon l'une des revendications 1 à 5, **caractérisé en ce que** le potentiel restant de biogaz dans un résidu de fermentation est acquis en tant que grandeur mesurée.

7. Procédé selon l'une des revendications 1 à 6, **caractérisé en ce que** l'unité de régulation (4) fait varier la vitesse de rotation de l'agitateur (2).

8. Procédé selon l'une des revendications 1 à 7, **caractérisé en ce que** l'unité de régulation (4) commute au moins un agitateur supplémentaire (11).

9. Procédé selon l'une des revendications 1 à 8, **caractérisé en ce que** l'unité de régulation (4) fait varier la quantité et/ou la composition du substrat acheminé et/ou d'une substance remise en circulation et/ou modifie de manière biochimique ou physique l'aptitude à l'écoulement du substrat de fermentation.

10. Dispositif de production de biogaz à partir de substances organiques, un substrat étant acheminé à un récipient (1) au moyen d'un système de distribution (13) et au moins un agitateur (2) étant disposé dans le récipient (1), **caractérisé en ce que** la valeur réelle d'au moins une grandeur mesurée est acquise et retransmise à une unité de régulation (4) et une valeur de consigne est stockée dans l'unité de régulation (4), notamment une valeur de consigne spécifique au fonctionnement qui dépend de la taille du récipient (1) et/ou de la nature des agitateurs et/ou de la position des agitateurs les uns par rapport aux autres, l'unité de régulation (4) calculant l'écart entre la valeur réelle et la valeur de consigne et, en fonction de cet écart, faisant varier des grandeurs de commande qui modifient la puissance consommée de l'agitateur (2) et/ou la composition du contenu du récipient et/ou l'aptitude à l'écoulement du contenu du récipient, notamment empêchent la formation de cavités dans la zone proche de l'hélice.

11. Dispositif selon la revendication 10, **caractérisé en ce que** le débit massique de gaz produit est acquis en tant que grandeur mesurée.

12. Dispositif selon la revendication 10 ou 11, **caractérisé en ce que** la part de gaz méthane est acquise en tant que grandeur mesurée.

13. Dispositif selon l'une des revendications 10 à 12, **caractérisé en ce que** les données de puissance d'une machine / d'un groupe qui traite le biogaz sont acquises en tant que grandeur mesurée.

14. Dispositif selon l'une des revendications 10 à 13, **caractérisé en ce que** l'étendue d'une couche flottante qui se forme est acquise en tant que grandeur mesurée.

15. Dispositif selon l'une des revendications 10 à 14, **caractérisé en ce que** le potentiel restant de biogaz dans un résidu de fermentation est acquis en tant que grandeur mesurée.

16. Dispositif selon l'une des revendications 10 à 15, **caractérisé en ce que** l'unité de régulation (4) fait varier la vitesse de rotation de l'agitateur (2).

17. Dispositif selon l'une des revendications 10 à 16, **caractérisé en ce que** l'unité de régulation (4) commute au moins un agitateur supplémentaire (11).

18. Dispositif selon l'une des revendications 10 à 17, **caractérisé en ce que** l'unité de régulation (4) fait varier la quantité et/ou la composition du substrat acheminé et/ou d'une substance remise en circulation et/ou modifie de manière biochimique ou physique l'aptitude à l'écoulement du substrat de fermentation.
